Europäisches Patentamt

European Patent Office

Office européen des brevets

(19)

(11) Veröffentlichungsnummer: **0 327 045**
**A1**

## (12) EUROPÄISCHE PATENTANMELDUNG

(21) Anmeldenummer: 89101691.7

(22) Anmeldetag: 01.02.89

(51) Int. Cl.⁴ **C12P 1/06 , C07G 11/00 ,
A61K 35/66 , //(C12P1/06,
C12R1:465)**

Der (Die) Mikroorganismus (Mikroorganismen) ist (sind) bei DSM unter der (den) Nummer(n) 4329 hinterlegt worden.

(30) Priorität: 05.02.88 DE 3803383

(43) Veröffentlichungstag der Anmeldung:
09.08.89 Patentblatt 89/32

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Franco, Christopher Milton Mathew, Dr.
74A, "The Westminister" N.S. Mankikar Marg
Sion Bombay 400 022(IN)
Erfinder: Chatterjee, Sugata, Dr.
H1/2 Hoechst Quarters Darga Road
Mulund (West) Bombay 400 080(IN)
Erfinder: VijayaKumar, Erra Koteswara Satya, Dr.
K-3, Hoechst Quarters Darga Road
Mulund (West) Bombay 400 080(IN)
Erfinder: Ganguli, Bimal Naresh, Dr.
"Saurayuth" 173 Central Avenue
Chembur Bombay 400 071(IN)
Erfinder: Rupp, Richard Helmut, Dr.
Roederweg 16A
D-6240 Königstein/Taunus(DE)

(54) **Ein neues Antibiotikum, Cammunocin, ein Verfahren zu seiner Herstellung und seine Verwendung als Arzneimittel.**

(57) Cammunocin, ein Wirkstoff, der durch Kultivierung von Streptomyces species Y-84,36210 (DSM 4329) gewonnen werden kann, besitzt antibiotische und immunsuppressive Wirksamkeit.

EP 0 327 045 A1

# Ein neues Antibiotikum, Cammunocin, ein Verfahren zu seiner Herstellung und seine Verwendung als Arzneimittel

Die vorliegende Erfindung betrifft ein neues Antibiotikum, das als Cammunocin bezeichnet wird, ein Verfahren zu dessen Herstellung aus Streptomyces spezies Y-84,36210 (hinterlegt bei der Deutschen Sammlung für Mikroorganismen am 23.12.1987 unter der Nr. DSM 4329), seinen Varianten und Mutanten, sowie die Verwendung von Cammunocin als Arzneimittel.

Str. sp. Y-84,36210 wurde aus einer bei Pune, Maharashtra, Indien gesammelten Bodenprobe isoliert. Varianten und Mutanten der Kultur Nr. HIL Y-84,36210 sind in bekannter Weise unter Verwendung eines Mutagens wie z.B. N-Methyl-N'-nitro-N-nitrosoguanidin oder ultraviolettem Licht erhältlich. Der Mikroorganismus Str. sp. Y-84,36210 gehört zur Ordnung der Actinomycetales, Familie Streptomycetaceae und Gattung Streptomyces.

Str. sp. Y-84,36210 wird als neuer Stamm angesehen, da er sich in einigen seiner morphologischen, Züchtungs- und physiologischen Eigenschaften von den bekannten Stämmen unterscheidet, wie aus der nachfolgenden Beschreibung ersichtlich ist. Ein weiterer Grund dafür, daß er als neuer Stamm betrachtet wird, ist daß er einen neuen, in der nachfolgenden Beschreibung charakterisierten, hier als Cammunocin bezeichneten antibiotischen Komplex produziert, der Gegenstand der vorliegenden Erfindung ist.

Wie aus der nachfolgenden detaillierten Beschreibung der Erfindung hervorgeht, ist das Cammunocin dieser Erfindung ein Peptid-Antibiotikum, unterscheidet sich jedoch von allen bekannten Peptid-Antibiotika wie Actinomycin, Viridogrisein, Valinomycin, Chinomycin, Cyclosporin, Polymyxin oder Amphomycin. Im Gegensatz zu den anderen bekannten Peptid- oder peptidhaltigen Antibiotika benötigt Cammunocin mindestens ca. 10 mM Calciumionen, um eine antibakterielle Wirkung zu entfalten. Dadurch unterscheidet es sich deutlich von anderen bekannten Antibiotika wie Amphomycin, Glumamycin, Zaomycin und dem A 21978 C-Komplex, welche mit 1,0 mM einen geringeren Bedarf an Calciumionen haben, um eine antibiotische Wirkung zu zeigen. Amphomycin, Glumamycin und Zaomycin gehören zu der Klasse der cyclischen Lipopeptid-Antibiotika, die im "CRC-Handbook of Antibiotic compounds [CRC-Handbuch antibiotischer Verbindungen]", Band IV, Teil 1, S. 313-327, Janos Berdy (Verfasser), CRC Press, Boca Raton, Florida (1980), aufgeführt sind. Ein A 21978 C-Komplex, ebenfalls ein cyclisches Lipopeptid-Antibiotikum, ist in J. Antibiotics 40, 761-777 (1987) beschrieben.

Gegenstand dieser Erfindung ist weiterhin ein Verfahren zur Herstellung des neuen antibiotischen Komplexes Cammunocin, welches dadurch gekennzeichnet ist, daß man Str. sp. Y-84,36210, seine Varianten und Mutanten, unter aeroben Bedingungen bei einer Temperatur vorzugsweise zwischen ca. 18 und 37°C in einem wäßrigen Nährmedium, das Kohlenstoff- und Stickstoffquellen und Mineralstoffe enthält, bei einem pH vorzugsweise zwischen ca. 6 und 9 züchtet und den antibiotischen Komplex aus dem Nährmedium gewinnt.

Das neue Antibiotikum dieser Erfindung ist wirksam in vitro gegenüber einer Reihe von gram-positiven Mikroorganismen, jedoch nur in Gegenwart von Calciumionen. Es ist ebenso wirksam als immunmodulierende Substanz und kann demzufolge in der Humanmedizin angewendet werden.

Gegenstand der vorliegenden Erfindung ist ferner ein Verfahren zur Isolierung von Str. sp. Y-84,36210 aus dem Boden, wobei eine Nährlösung vorzugsweise mit einem pH zwischen ca. 6,5 und 8,5 in bekannter Weise verwendet wird.

Die für die Isolierung der Mikroorganismen aus dem Boden verwendete Nährlösung besteht aus Kohlenstoff- und Stickstoffquellen, anorganischen Nährsalzen und Verfestigungsmitteln. Als Kohlenstoffquelle kommen vorzugsweise z.B. Glukose, Stärke, Dextrin, Glycerin, Saccharose oder Melasse in Frage. Bevorzugte Stickstoffquellen sind Pepton, Hefeextrakt, Rindfleischextrakt, Malzextrakt, Casein oder Aminosäuren, wie Arginin oder Asparagin. Als Verfestigungsmittel kann zum Beispiel Agar verwendet werden. Als anorganische Nährsalze kommen vorzugsweise Natrium-, Kalium-, Magnesium- oder Calciumsalze des Phosphors oder Schwefels in Betracht.

Der erfindungsgemäße Mikroorganismus verlängert farblose Luftmycelien aus verzweigten Substratmycelien. Auf den Luftmycelien werden spiralförmige Sporenketten gebildet. Die Spiralen sind offen. Kurze, den Abschnitt RA repräsentierende Sporenketten sind ebenfalls häufig. Es wird weder Wirbel- noch Ascosporenbildung beobachtet. Reife Sporenketten besitzen 30 - 50 Sporen pro Kette. Die Eigenschaften des Mikroorganismus bei der Kultur auf verschiedenen Agarmedien wird wie folgt beschrieben (vgl. Oxoid-Handbuch 1972, 2. Auflage, Herausgeber Oxoid Limited, London, England oder Difco Manual, 9. Auflage 1977, Herausgeber Difco Laboratories, Detroit, Michigan, USA):

| 1. Hefeextrakt - Malzextraktagar | | |
|---|---|---|
| Wachstum | : | gut, faltig, trocken |
| Luftmycel | : | gut, pulverig, schwach blaugrau |
| Rückseite | : | schwarz-purpurn |
| Lösliches Pigment | : | braun-purpurn |

| 2. Hafermehlagar | | |
|---|---|---|
| Wachstum | : | reichlich, flach, trocken |
| Luftmycel | : | reichlich, pulverig, hellgrau |
| Rückseite | : | dunkelbraun-purpurn |
| Lösliches Pigment | : | braun-purpurn |

| 3. Anorganische Salze - Stärkeagar | | |
|---|---|---|
| Wachstum | : | reichlich, erhöht, trocken |
| Luftmycel | : | gut, pulverig, helles blaugrau |
| Rückseite | : | dunkles schwarzviolett |
| Lösliches Pigment | : | hell malvenfarbig |

| 4. Glycerin - Asparaginagar | | |
|---|---|---|
| Wachstum | : | gut, erhöht, trocken |
| Luftmycel | : | gut, pulverig, graurosa |
| Rückseite | : | schwarz-purpurn |
| Lösliches Pigment | : | helles purpurbraun |

| 5. Pepton - Hefeextrakt - Eisenagar | | |
|---|---|---|
| Wachstum | : | gut, faltig, feucht |
| Luftmycel | : | keines |
| Rückseite | : | schwach gelb |
| Lösliches Pigment | : | schwach braun |

| 6. Tyrosinagar | | |
|---|---|---|
| Wachstum | : | reichlich, faltig, trocken |
| Luftmycel | : | reichlich, pulverig, schwach graublau |
| Rückseite | : | dunkles schwarzviolett |
| Lösliches Pigment | : | schwach braunviolett |

| 7. Saccharose - Nitratagar | | |
|---|---|---|
| Wachstum | : | reichlich, flach, trocken |
| Luftmycel | : | gut, pulverig, schwaches blaugrau |
| Rückseite | : | schwach gelb |
| Lösliches Pigment | : | schwach violett |

| 8. Pepton - Rindfleischextraktagar (Nähragar) | | |
|---|---|---|
| Wachstum | : | mäßig, erhöht, trocken |
| Luftmycel | : | schwach, pulverig, dunkelgrau |
| Rückseite | : | graurosa |
| Lösliches Pigment | : | - |

Das lösliche Pigment ist ein pH-Wert-Indikator: es wird rosarot in saurem Milieu und blauviolett im alkalischen Bereich.

Der optimale Wachstumstemperaturbereich für den erfindungsgemäßen Mikroorganismus liegt zwischen ca. 25 und 35°C. Der Mikroorganismus verflüssigt Gelatine in Glukose-Pepton-Gelatinemedium, hydrolysiert Stärke in anorganischem Salz-Stärkeagar und koaguliert Magermilch. Str. sp. Y-84,36210 wächst gut auf Czapek's Agarlösung (vgl. Oxoid-Handbuch).

Eine sehr schwache Bildung von dunklem Pigment wird nur in Tyrosinagar beobachtet, wobei keine Pigmentbildung in Pepton-, Hefeextrakt-, Eisenagar- oder Trypton-Hefeextraktbouillon auftritt.

Das Kohlenstoffquellen-Assimilationsschema dieses Mikroorganismus ist wie folgt (in Pridham-Gottlieb-Medium):

Positiv: D-Glukose, L-Arabinose, D-Xylose, I-Inosit, D-Mannit, D-Fruktose, Rhamnose, Galaktose, Maltose, Cellobiose, Na-Glutamat, Mannose, Lactose

Fraglich: Saccharose, Salicin

Negativ: Raffinose, Cellulose, Dulcit

Str. sp. Y-84,36210 wird durch Streptomycin in Konzentrationen größer als 1,6 $\mu$g/ml gehemmt, verträgt NaCl-Konzentrationen von ca. 7 - 10 % und hat einen pH-Toleranzbereich von ca. 5,5 - 9,0.

Die veröffentlichten Angaben zu den Züchtungs- und physiologischen Eigenschaften bekannter Mikroorganismen zeigen deutliche Unterschiede im Vergleich zu dem erfindungsgemäßen Mikroorganismus.

Darüberhinaus erzeugt Str. sp. Y-84,36210, wenn es fermentiert wird, den neuen antibiotischen Komplex Cammunocin.

Ausgehend von den obengenannten Beobachtungen kann der erfindungsgemäße Mikroorganismus als neue Streptomyces-Art angesehen werden.

Es ist für den Fachmann selbstverständlich, daß diese Erfindung nicht auf den speziellen, oben definierten Organismus beschränkt ist, sondern alle solchen spontanen und künstlichen, von dem besagten Mikroorganismus abgeleiteten Mutanten und Varianten einschließt, die die Fähigkeit haben, den neuen antibiotischen Komplex Cammunocin zu bilden.

Gegenstand der vorliegenden Erfindung ist ferner ein Verfahren zur Herstellung von Cammunocin, welches dadurch gekennzeichnet ist, daß man Str. sp. Y-84,36210 durch Fermentation vorzugsweise bei einem pH-Wert zwischen ca. 6,0 und 9,0 und vorzugsweise bei einer Temperatur zwischen ca. 18 - 37°C unter aeroben Bedingungen in einem Nährmedium züchtet, das Kohlenstoff- und Stickstoffquellen, anorganische Nährsalze und Spurenelemente enthält, und die Verbindungen aus der Kulturbrühe in bekannter, wie hier beschriebener Weise isoliert.

Als Kohlenstoffquellen für das zur Herstellung der neuen Antibiotika verwendete Nährmedium kommen bevorzugt z.B. Glukose, Stärke, Dextrin, Glycerin, Saccharose, Melasse oder Öl in Frage. Bevorzugt geeignete Stickstoffquellen im Nährmedium für die Herstellung der neuen Antibiotika sind Sojabohnenmehl, Hefeextrakt, Rindfleischextrakt, Malzextrakt, Maisquellwasser, Pepton oder Casein. Für die Verwendung im Nährmedium für die Herstellung der neuen Antibiotika in Betracht kommende anorganische Nährsalze/Mineralsalze sind vorzugsweise Natriumchlorid, Magnesiumsulfat, Ammoniumsulfat oder Calciumcarbonat. Als Spurenelemente werden Eisen, Mangan, Kupfer, Zink oder Kobalt bevorzugt verwendet.

Bei einer bevorzugten Ausführungsform der vorliegenden Erfindung wird Str. sp. Y-84,36210 bei ca. 26-28°C und pH ca. 6,4 - 6,6 gezüchtet. Nach ca. 40 - 45 Stunden dauernder Fermentation erhält man die höchsten Ausbeuten der Verbindungen. Bei der Fermentation handelt es sich vorzugsweise um eine Submersfermentation. Der Fermentationsverlauf und die Bildung des neuen antibiotischen Komplexes lassen sich anhand der antibakteriellen Wirksamkeit der Kulturflüssigkeit und des Mycels gegenüber Staphylococcus aureus 209 P in 30 mM Calciumchlorid enthaltendem Agarmedium verfolgen.

Gegebenenfalls kann dem Nährmedium während der Fermentation der Kultur ein Schaumverhütungsmittel wie zum Beispiel Desmophen[R] (Polyole der Firma Bayer AG, Leverkusen) zugegeben werden.

Cammunocin kann aus der Kulturbrühe z.B. durch direkte Adsorption an geeigneten Adsorptionsmitteln oder durch Lösungsmittelextraktion und nachfolgende Adsorption gewonnen werden. Bevorzugte Lösungsmittel sind z.B. Mischungen von Ethylacetat oder Chloroform mit n-Propanol; besonders bevorzugt ist eine Ethylacetat/n-Propanol Mischung (2:1). Beispielsweise kann das Kulturfiltrat oder der die erfindungsgemäße

4

Verbindung enthaltende Lösungsmittelextrakt des Kulturfiltrats an Aktivkohle, polymeren Adsorptionsmitteln wie z.B. Diaion[(R)] HP-20 (Mitsubishi Chemical Industries, Japan) oder Amberlite[(R)] XAD (polymeres Adsorptionsmittel aus einer Matrix aus Polystyrol,Acrylester oder Aminoxid mit einem durchschnittlichen Porendurchmesser von $(40-225) \cdot 10^{-10}$ m, Rohm & Haas Co., USA) adsorbiert werden. Der Lösungsmittelextrakt wird vorzugsweise zur Entfernung des Lösungsmittels eingeengt und anschließend über das Adsorptionsmittel chromatographiert. Die erfindungsgemäße Verbindung kann unter Verwendung geeigneter mobiler Phasen wie z.B. Chloroform, Methanol oder Aceton bzw. unter Verwendung von Gemischen dieser Lösungsmittel untereinander oder mit Wasser aus den Adsorptionsmitteln eluiert werden, und die Eluate können anschließend zur Trockne eingedampft werden. Das vorzugsweise verwendete Elutionsmittel ist Methanol.

Cammunocin kann auch unter Anwendung von Ionenaustauscherchromatographie aus dem Kulturfiltrat isoliert werden. Geeignete Harze sind z.B. Anionenaustauscher-Harze des schwach basischen Polystyrol-, Polyamin- oder vernetzten Aminoalkoholpolyacrylestertyps wie z.B. Dowex[(R)] (Dow Chemical Company, USA) oder Amberlite[(R)] IRA 68 (Rohm & Haas Co., USA). Der vorzugsweise verwendete Ionenaustauscher ist Amberlite[(R)] IRA 68 (Acryl-Typ, Tertiäres Amin-Funktionalität). Bei diesem Verfahren wird das Kulturfiltrat vorzugsweise einer Säulenchromatographie unterzogen, wobei das Anionenaustauscher-Harz Amberlite[(R)] IRA 68 (Cl$^{-}$) verwendet wird. Die erfindungsgemäße Verbindung wird zunächst vom Ionenaustauscher adsorbiert und anschließend unter Verwendung geeigneter mobiler Phasen, wie z.B. wäßriger oder methanolischer Natrium- oder Kaliumchloridlösungen, verdünnter Salzsäure- oder Natriumhydroxidlösungen, eluiert, wobei vorzugsweise eine 2 m NaCl-Lösung verwendet wird. Die aktiven Eluate können vereinigt und unter Verwendung der obengenannten Adsorptionschromatographie entsalzt werden. Die so gewonnenen entsalzten, aktiven Eluate werden gesammelt und konzentriert.

Die zuvor genannten konzentrierten Cammunocin-haltigen Eluate können auf verschiedene Weise weiter gereinigt werden. Zum Beispiel können Readsorption und Elution mit Aktivkohle, polymeren Adsorptionsmitteln wie z.B. Amberlite[(R)] XAD-4 (bestehend aus einer Matrix aus Polystyrol, durchschnittlicher Porendurchmesser $40 \cdot 10^{-10}$ m) und 7 (bestehend aus einer Matrix aus Acrylester, durchschnittlicher Porendurchmesser $90 \cdot 10^{-10}$ m, Rohm & Haas Co., USA), Diaion[(R)] HP-20 (Mitsubishi Chemical Industries, Japan)-,Gelfiltration mit lipophilen Gelfiltrationsmaterial wie z.B. Sephadex[(R)] LH-20 und G-Serien-Gelen (Pharmacia Fine Chemicals AB, Schweden) und gleichwertigen Produkten sowie Ionenaustauschgelfiltration mit Gelen mit Diethylaminoethyl (DEAE)-Funktionalität wie z.B. Sephadex[(R)] DEAE-Gelen (Pharmacia Fine Chemicals AB, Schweden), Adsorptionschromatographie auf Aluminiumoxid und Kieselgel zur weiteren Reinigung gut miteinander kombiniert werden. Zudem können auch Dünnschichtchromatographie, Mitteldruck- und Hochdruck-Flüssigkeitschromatographie mit geeigneten Adsorptionsmitteln wie Kieselgel und modifiziertem Kieselgel-C$_{18}$ (erhältlich z.B. durch Reaktion von Silikagel mit Octadecyltrichlorosilan) und geeigneten Elutionsmitteln verwendet werden. Weiterhin kann Gegenstromchromatographie mit einem bestimmten Lösungsmittelsystem den besagten Zweck gut erfüllen.

Cammunocin ist ein farbloses, amorphes Pulver, das in Wasser, Methanol, Ethanol, Propylenglykol und Dimethylsulfoxid löslich ist. Es ist schwer- oder unlöslich in Aceton, Methylenchlorid, Ethylacetat, Chloroform, Hexan und Petrolether (40-60°). Es zeigt im Ninhydrinfarbtest eine negative Reaktion.

In den unten angegebenen dünnschichtchromatographischen (DC) Systemen hat Cammunocin die folgenden Rf Werte:

DC-Platte: Fertigplatte mit Kieselgel, Artikel Nr. 5554 von E. Merck, Darmstadt.

| | EtOAc:MeOH:H$_2$O | Butanol:AcOH:H$_2$O |
|---|---|---|
| | 4 : 4 : 1 | 4 : 4 : 1 |
| Cammunocin Rf | 0,47 | 0,39 |

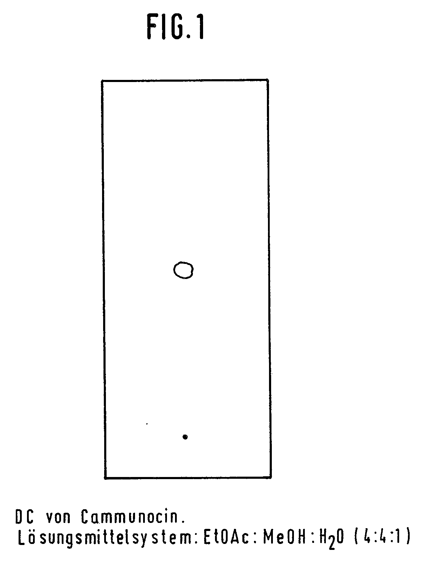

Figur 1 zeigt das bei der DC verwendete Fließmittel: EtOAc:MeOH:H$_2$O (4:4:1). Nachweis bei 254 nm.
Figur 2 zeigt die analytische Hochdruckflüssigkeitschromatographie (HPLC). Die HPLC wurde wie folgt durchgeführt:

| Säulenfüllung | : | ODS-Hypersil[R] (10 µ, 4x120mm) (HPLC-Material mit Octadecyltrichlorosilan-Gruppen der Firma Shandon, USA) |
| Durchflußgeschwindigkeit | : | 0,5 ml/Minute |
| Nachweis | : | bei 234 nm |
| Lösungsmittel | : | MeOH:1 %ige wäßrige Essigsäure (55:45) |

Cammunocin schmilzt bei 270° C unter Zersetzung.

Die spektroskopischen Daten für Cammunocin sind nachstehend angeführt:

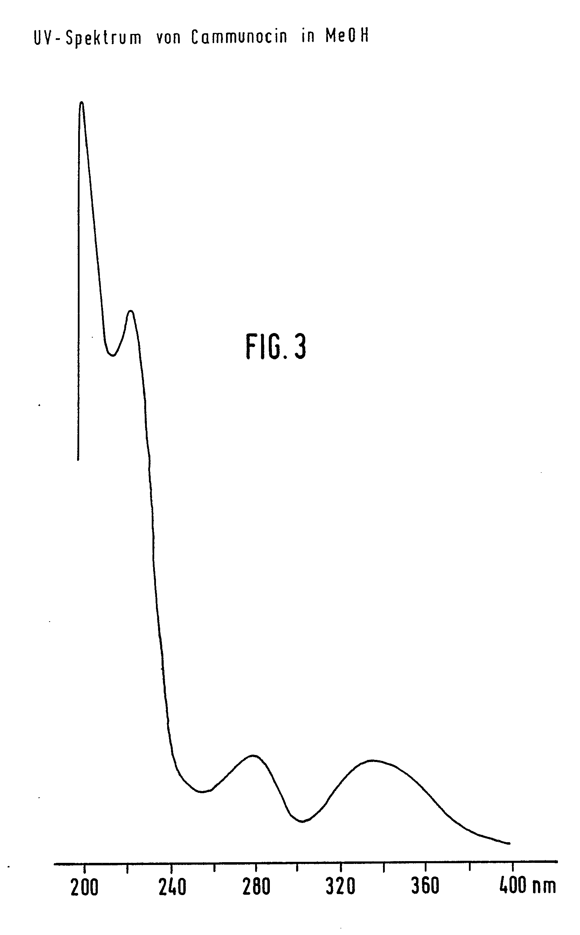

1. UV max in Methanol liegt bei ca. 224, 280, 345 nm - siehe Figur 3. Die UV-Absorptionsmaxima des Cammunocins wurden in einer Konzentration von 0,2 g pro Liter bestimmt. Das Absorptionsspektrum wurde mit einem Uvikon 810 Spektrophotometer im Bereich von 200 bis 800 nm gemessen.

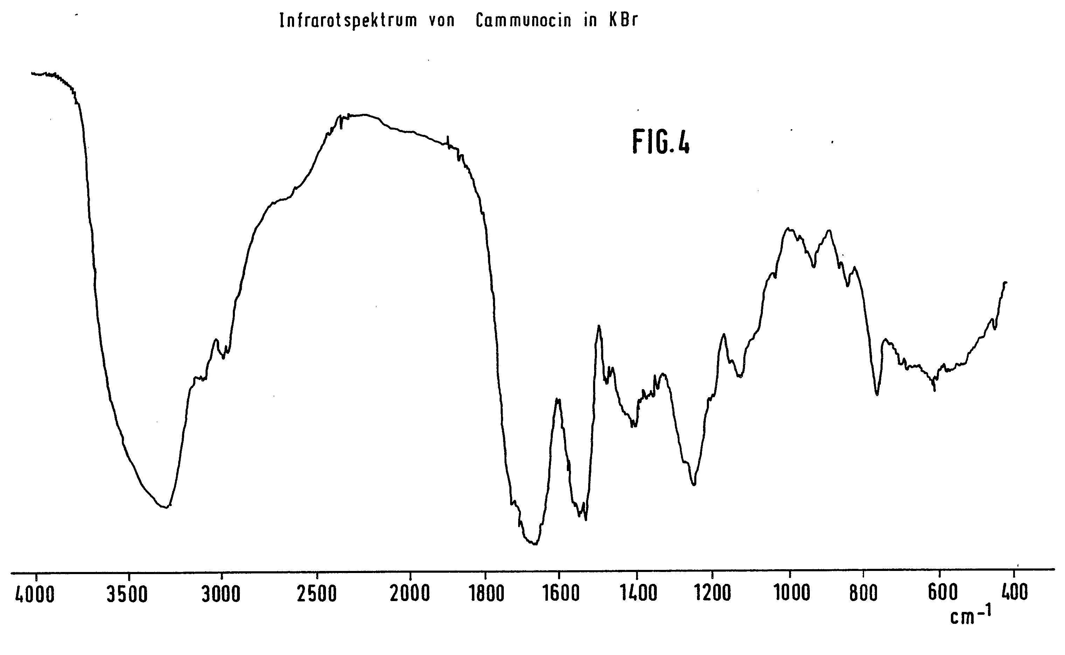

2. Das IR-Spektrum (KBr-Tablette) wurde mit einem Perkin Elmer-Spektrometer P.E. 521 gemessen - siehe Figur 4.

Die oben genannten spektroskopischen Daten und die in Beispiel VI beschriebenen chemischen und spektroskopischen Analysen deuten darauf hin, daß Cammunocin ein Komplex miteinander verwandter Peptid-Antibiotika ist.

Ein einmaliges Charakteristikum des Cammunocins ist, daß es seine antibiotische Wirkung gegen gram-positive Bakterien nur in Gegenwart von Calciumionen entfaltet. Andere Metallionen, wie z.B. Natrium, Kalium, Barium, Rubidium und Magnesium haben nicht dieselbe Wirkung. Deshalb wird Cammunocin in einem Agarmedium getestet, das mindestens ca. 10 mM Calciumchlorid enthält.

In dem obengenannten, mit Calcium angereicherten Agar zeigt Cammunocin in vitro eine Wirkung gegenüber empfindlichen und resistenten Staphylococcus aureus und Streptomyces faecalis-Stämmen wie gegenüber Bacillus subtilis, Sarcina lutea, Streptococcus pyogenes und Micrococcus luteus. Die minimale Hemmkonzentration (MHK) des Cammunocins gegenüber verschiedenen Mikroorganismen wurde bestimmt. Die Ergebnisse sind in der nachfolgenden Tabelle 1 aufgeführt:

Tabelle 1

| MHK von Cammunocin | | | | | |
|---|---|---|---|---|---|
| Testmedium: Müller-Hinton-Agar (vgl. Oxoid-Handbuch) mit Zusatz verschiedener $Ca^{2+}$-Konzentrationen. | | | | | |
| Testorganismus | $Ca^{2+}$-Konzentrationen | | | | |
| | 0,0mM | 2,5mM | 10,0mM | 30,0mM | 60,0mM |
| S.aureus 209 P | >100,0 | 25,0 | 6,3 | 1,6 | <0,1 |
| S.aureus 20464 Mak[R] | >100,0 | 100,0 | 25,0 | 1,6 | 1,6 |
| S.aureus 3066 Met[R] | >100,0 | 50,0 | 6,3 | 6,3 | 0,8 |
| S.aureus R 85, Em[R] | >100,0 | >100,0 | >50,0 | 25,0 | 6,3 |
| S.aureus R 85/M,Em[R] | >100,0 | >100,0 | >50,0 | 2,5 | 1,6 |
| S.aureus 712 Met[R] | >100,0 | 100,0 | 6,3 | 6,3 | 0,4 |
| S.aureus 789 Met[R] | >100,0 | 100,0 | 6,3 | 1,6 | 0,8 |
| Str.faecalis UD86 | >100,0 | 100,00 | 25,0 | 6,3 | 0,8 |
| Str.faecalis Eder Mak[R] | >100,0 | 100,0 | 25,0 | 6,3 | 1,6 |
| S.aureus MLS 11 | >100,0 | >100,0 | 12,5 | 3,2 | 1,6 |
| S.aureus MLS 14 | >100,0 | 50,0 | 25,0 | 12,5 | 1,6 |
| S.aureus MLS 16 | >100,0 | 100,0 | 25,0 | 25,0 | 1,6 |
| S.aureus O11UC5 | >100,0 | 100,0 | >50,0 | 12,5 | 1,6 |
| S.aureus 011GR5 | >100,0 | 100,0 | >50,0 | >50,0 | NT |
| Str.faecalis 02 | >100,0 | 100,0 | 25,0 | 6,3 | 1,6 |
| Micrococcus luteus | >100,0 | 3,2 | 0,8 | 0,2 | <0,1 |
| Sarcina lutea | >100,0 | 25,0 | 6,3 | 1,6 | 0,8 |
| Bacillus subtilis | >100,0 | 100,0 | 6,3 | 3,2 | 0,8 |
| E. coli 9632 | >100,0 | >100,0 | >100,0 | >100,0 | >100,0 |
| E.coli 2231 | >100,0 | >100,0 | >100,0 | >100,0 | >100,0 |
| Candida albicans | >100,0 | >100,0 | >100,0 | >100,0 | >100,0 |
| Aspergillus niger | >100,0 | >100,0 | >100,0 | >100,0 | >100,0 |
| Mak[R] = resistent gegen Makrolid-Antibiotika>100,0<br>Met[R] = resistent gegen Meticillin<br>Em[R] = resistent gegen Erythromycin | | | | | |

## Immunmodulierende Wirkung

Die immunmodulierende Wirkung von Cammunocin wurde wie folgt geprüft:

## Hämolyse-Plaque-Test (PFC):

Weibliche Swiss-Mäuse mit einem Gewicht von 16 - 18 g (6 pro Gruppe) werden durch intraperitoneale Injektion von Schaferythrozyten ($5 \times 10^8$ Zellen) sensibilisiert. Nach 5 Tagen werden die Mäuse durch Genickbruch getötet, die Milz wird entfernt und in Dulbecco-Lösung eisgekühlt gelagert. Die Splenozyten werden durch vorsichtiges Zerzupfen der Milz auf einem feinmaschigen Drahtgitter gewonnen. Ihre Lebensfähigkeit wird bestimmt und ihre Menge auf ca. $6 \times 10^6$ Zellen/ml eingestellt. Anschließend werden die Splenozyten mit Schaferythrozyten in Gegenwart von Komplement in einer Cunningham-Kammer bei $37°$ C unter 5 %igem $CO_2$ zur Reaktion gebracht. Die Plaquen, die sich gebildet haben, werden nach 2 Stunden gezählt.

Cammunocin wurde vom Tage der Sensibilisierung an täglich in Dosierungen von je 5, 10 und 20 mg/kg bzw. 10 mg/kg intraperitoneal bzw. subkutan injiziert. Die letzte Dosis der Verbindung wurde am 5. Tag, eine Stunde vor Tötung der Tiere, verabreicht. Die Ergebnisse sind in Tabelle 2 aufgeführt.

Tabelle 2

| Dosis mg/kg, x 5 | Applikations weg | Prozentuale Hemmung der PFC |
|---|---|---|
| 5,0 | i.p. | 31,20 |
| 10,0 | i.p. | 40,66 ± 4,67 |
| 20,0 | i.p. | 41,4 ± 4,89 |
| 10,0 | s.c. | 38,4 |

Diese Ergebnisse zeigen, daß Cammunocin eine immunsuppressive Wirkung hat und somit als Immunosuppressivum z.B. bei Transplantationen eingesetzt werden kann.

Die pharmakologischen Eigenschaften qualifizieren die erfindungsgemäßen Verbindungen für den Einsatz als Therapeutikum. Erfindungsgegenstand sind demzufolge auch Arzneimittel mit einem Gehalt an Cammunocin neben üblichen, allgemein bekannten Hilfs- und/oder Trägerstoffen, sowie die Verwendung von Cammunocin zur Herstellung von Arzneimitteln mit antibiotischer und/oder immunsuppressiver Wirkung in an sich bekannter Weise.

Im folgenden soll die Erfindung anhand einiger bevorzugter Beispiele näher erläutert werden, doch soll sie nicht als auf diese Beispiele beschränkt betrachtet werden.

**Beispiel 1**

**Isolierung von Streptomyces sp. Y-84,36210 aus Boden**

(a) Herstellung der Isolierungsnährmedien

| Medium 1: | |
|---|---|
| Glukose | 1,0 g |
| Glycerin | 1,0 g |
| L-Arginin | 0,3 g |
| $K_2HPO_4$ | 0,3 g |
| $MgSO_4 \cdot 7H_2O$ | 0,2 g |
| NaCl | 0,3 g |
| Hefeextrakt | 0,2 g |
| $Fe_2(SO_4)_3$ | 10,0 mg |
| $CuSO_4 \cdot 5H_2O$ | 1 mg |
| $ZnSO_4 \cdot 7H_2O$ | 1 mg |
| $MnSO_4 \cdot 7H_2O$ | 1 mg |
| Agar | 15,0 g |
| Destilliertes Wasser | 1 l |
| pH | 6,5 |

| Medium 2: | |
|---|---|
| Glukose | 2,0 g |
| L-Asparagin | 1,0 g |
| $K_2HPO_4$ | 0,5 g |
| $MgSO_4 \cdot 7H_2O$ | 0,5 g |
| Bodenextrakt | 200 ml |
| Agar | 15,0 g |
| Destilliertes Wasser | 800 ml |
| pH | 8,0 |

| Medium 3: | |
|---|---|
| Stärke | 10,0 g |
| Kasein | 0,3 g |
| $KNO_3$ | 2,0 g |
| NaCl | 2,0 g |
| $K_2HPO_4$ | 2,0 g |
| $MgSO_4 \cdot 7H_2O$ | 0,05 g |
| $CaCO_3$ | 0,02 g |
| $FeSO_4$ | 0,01 g |
| Agar | 15,0 g |
| Destilliertes Wasser | 1 l |
| pH | 7,2-7,5 |

Die Medien werden 30 Minuten lang bei 121°C sterilisiert. Jedesmal läßt man die sterilisierten Medien auf 45°C abkühlen, füllt sie in Petrischalen und läßt sie fest werden.

### (b) Herstellung der Bodensuspension

1 g Boden wird 1 Stunde lang im Heißluftofen bei 110°C erhitzt. Nach dem Abkühlen wird der Boden in destilliertem Wasser suspendiert und gut umgeschüttelt. Man läßt den Boden sich absetzen und verwendet den Überstand zur Impfung von jedem einzelnen der oben genannten Isolierungsnährmedien.

### (c) Impfung des Isolierungsmediums

1 ml der Bodensuspension wird auf Petrischalen überimpft, die jeweils 50 ml eines beliebigen der obengenannten Isolierungsmedien enthalten.

### (d) Isolierung von Streptomyces sp. Y-84,36210

Die beimpfte Petrischalen wird 10 Tage lang bei 37°C inkubiert, und Streptomyces sp. Y-84,36210 wird aus den wachsenden Mikroorganismen isoliert.

### Beispiel 2

### Züchtung von Streptomyces sp. Y-84,36210 zur fermentativen Herstellung von Cammunocin

Streptomyces sp. Y-84,36210 wird auf Hefe-Malz-Agar der folgenden Zusammensetzung gezüchtet:

| | |
|---|---|
| Malzextrakt | 10,0 g |
| Hefeextrakt | 4,0 g |
| Glukose | 4,0 g |
| Agar | 15,0 g |
| Destilliertes Wasser | 1 l |
| pH | 7,0 |

Das Medium wird auf Reagenzgläser verteilt und 30 Minuten lang bei 121°C sterilisiert. Die Reagenzgläser werden zur Herstellung von Schrägagars in Schrägstellung gekühlt. Die Schrägagars werden mit der Kultur geimpft und 10 bis 15 Tage lang bei 28°C inkubiert, wonach gutes Wachstum und Sporenbildung beobachtet werden. Eine Suspension der Sporen aus einem Schrägagar in destilliertem Wasser wird zur Beimpfung von 5 jeweils 100 ml des Impfkulturmediums enthaltenden 500 ml-Erlenmeyerkolben oder einer 1 l desselben Impfkulturmediums enthaltenden 5 l-Saugflasche verwendet.

## Zusammensetzung des Impfkulturmediums

| | |
|---|---|
| Glukose | 15,0 g |
| Sojabohnenmehl | 15,0 g |
| Maisquellwasser | 5,0 g |
| CaCO$_3$ | 2,0 g |
| NaCl | 5,0 g |
| Destilliertes Wasser | 1 l |
| pH | 6,5 |

Das obengenannte Medium wird jeweils in 100 ml-Portionen auf 500 ml-Erlenmeyerkolben oder in 1 l-Portionen auf 5 l-Saugflaschen verteilt und 30 Minuten lang bei 120°C sterilisiert. Die Kolben/Flaschen werden gekühlt, mit der Sporensuspension beimpft und bei 240 U.p.M 72 Stunden lang bei 27°C (±1°C) auf einer Rotationsschüttelmaschine mit 3,8 cm (1,5 Zoll) Ausschlag geschüttelt. Das so gewachsene Produkt wird zur Beimpfung von zwei 10 l 8 - 10 vol.-%iges Impfkulturmedium enthaltenden 15 l-Glasfermentern zur Herstellung der 2. Stufe der Impfkultur verwendet. Die Fermentation wird bei 27°C (±1°C) unter Rühren bei 180 - 200 U.p.M. und einer Belüftungsmenge von 6 - 7 lpm über einen Zeitraum von 24 Stunden durchgeführt. Die so erhaltene, gut gewachsene 2. Stufe der Impfkultur wird zur Beimpfung des Herstellungsmediums verwendet.

## Zusammensetzung des Herstellungsmediums

| | |
|---|---|
| Glukose | 15,0 g |
| Lösliche Stärke | 20,0 g |
| Sojaton | 3,0 g |
| Pepton | 3,0 g |
| CaCO$_3$ | 2,0 g |
| NaCl | 2,0 g |
| Maisquellwasser | 2,0 g |
| (NH$_4$)$_2$SO$_4$ | 0,5 g |
| Destilliertes Wasser | 1 l |
| pH | 6,5 |

Der Fermenterinhalt wird mit 0,025 % Desmophen als Entschäumungsmittel versetzt.

280 l des obigen Mediums werden in einem 390 l-Fermentationsgefäß vorgelegt. Das Medium wird 28 Minuten lang bei 121°C durch indirekten oder direkten Dampf sterilisiert. Das Fermentationsgefäß wird gekühlt und mit der 2. Stufe der Impfkultur (7 Vol.-%) beimpft. Die Fermentation erfolgt bei 27°C (±1°C) unter Rühren bei 100 - 120 U.p.M. Die Belüftungsmenge beträgt 170 l pro Minute. Bei Beendigung der Fermentation nach 40 - 45 Stunden beträgt der Durchmesser der Hemmzone gegen Staphylococcus aureus 209 P 20 mm bei der Prüfung des Kulturfiltrates nach der Agarnäpfchenmethode (6 mm Durchmesser) unter Verwendung von mit Calcium versetztem (30 mM) Agar bei einem pH-Wert der Kulturflüssigkeit im Bereich von 6,5 bis 6,9.

Das abgesetzte Zellvolumen beträgt 20 %.

Die geerntete, den antibiotischen Komplex enthaltende Kulturbrühe wird zur Trennung des Myzels von der Kulturflüssigkeit zentrifugiert und wie in Beispiel 4 beschrieben weiter verarbeitet.

**Beispiel 3**

**Züchtung von Streptomyces sp. Y-84,36210 zur fermentativen Herstellung von Cammunocin**

Das in Beispiel 2 beschriebene Verfahren wird unter folgenden Bedingungen wiederholt:

Str. sp. Y-84,36210 wird auf einem Agarmedium mit der folgenden Zusammensetzung gezüchtet:

| | |
|---|---|
| Stärke (löslich) | 10,0 g |
| $K_2HPO_4$ | 1,0 g |
| $MgSO_4 \cdot 7H_2O$ | 1,0 g |
| NaCl | 1,0 g |
| $(NH_4)_2SO_4$ | 2,0 g |
| $CaCO_3$ | 2,0 g |
| $FeSO_4 \cdot 7H_2O$ | 0,1 mg |
| $MoCl_2 \cdot 4H_2O$ | 0,1 mg |
| $ZnSO_4 \cdot 7H_2O$ | 0,1 mg |
| Agar | 15,0 g |
| Destilliertes Wasser | 1 l |
| pH | 7,2 |

Die Zusammensetzung des Impfkulturmediums gleicht der in Beispiel 2 beschriebenen.

**Zusammensetzung des Herstellungsmediums**

| | |
|---|---|
| Glukose | 20,0 g |
| Sojabohnenmehl | 10,0 g |
| $CaCO_3$ | 0,2 g |
| $CoCl_2 \cdot 6H_2O$ | 1,0 mg |
| Destilliertes Wasser | 1 l |
| pH | 7,0 |

100 l des obigen Mediums werden in einem 150 l-Fermentationsgefäß vorgelegt. Das Medium wird 28 Minuten lang bei 121°C durch indirekten oder direkten Dampf sterilisiert. Das Fermentationsgefäß wird abgekühlt und mit der 2. Stufe der Impfkultur (9 Vol.-%) beimpft. Die Fermentation erfolgt bei 27°C (±1°C) unter Rühren bei 80 - 90 U.p.M. mit einer Belüftungsmenge von 60 - 70 l pro Minute. Bei Beendigung der Fermentation nach 40 - 45 Stunden beträgt der pH der Kulturbrühe 6,45 und der Durchmesser der Hemmzone gegen Staphylococcus aureus 209 P 22 mm bei der Prüfung des Kulturfiltrats nach der Agarnäpfchenmethode (6 mm Durchmesser) unter Verwendung von mit Calcium versetztem (30 mM) Agar. Das abgesetzte Zellvolumen beträgt 12 Vol.-%. Die Kulturbrühe wird wie in Beispiel 5 beschrieben weiter verarbeitet.

**Beispiel 4**

11

240 ml des nach Beispiel 2 erhaltenen Kulturfiltrats werden auf eine 6 l des Anionenaustauscher-Harzes Amberlite[R] (IRA 68 (Cl⁻) (Austauscherharz mit Polystyrol-Polyamin-Funktionalität) enthaltende Säule aufgebracht. Nach Spülung mit 40 l entmineralisiertem Wasser wird die Säule mit wäßriger 2,0 m NaCl-Lösung, pH 8,5 (mit Ammoniak eingestellt), eluiert. Die erhaltenen aktiven Eluate (30 l) werden 3mal mit einer Ethylacetat:n-Propanol-Mischung 2:1 extrahiert, nachdem der pH mit HCl auf 4,0 eingestellt worden ist. Die Extrakte werden im Vakuum eingeengt und das so erhaltene Konzentrat (6,1 g) wird einer Kieselgelchromatographie (Siebgröße 0,062-0,037 mm (230 - 400 mesh, 600 g) unterzogen und über einen Gradienten von Chloroform bis Chloroform:Methanol (1:1) eluiert. Die vereinigten eingeengten aktiven Fraktionen (3 g) werden mit einer gesättigten wäßrigen NaHCO₃-Lösung extrahiert und dann wird der pH des Extraktes mit HCl auf 4,0 eingestellt, wonach eine erneute Extraktion mit einer Ethylacetat:n-Propanol-Mischung 2:1 erfolgt. Der Extrakt wird im Vakuum eingeengt, was 1,8 g der Verbindung ergibt, die dann einer Kieselgelchromatographie (Siebgröße 0,062-0,037 mm (230-400 mesh), 360 g) unterzogen wird. Elution erfolgt über einen Gradienten von Ethylacetat bis Ethylacetat:Methanol (1:1) und erbringt 860 mg aktives Material beim Einengen. Dieses Material wird in 6 Portionen aufgeteilt und getrennt auf 50 g Sephadex[R] LH-20 (lipophiles Gelfiltrationsmaterial) in Methanol enthaltende Säulen aufgetragen; als Elutionsmittel wird Methanol verwendet. Auf diese Weise werden 360 mg Cammunocin gewonnen.

## Beispiel 5

Die Kulturfiltrate aus den zwei Fermenterchargen gemäß Beispiel 3 werden vereinigt, so daß man ein Volumen von 185 l erhält. Diese Menge wird auf eine 4 l Amberlite[R] IRA-68 (Cl⁻) enthaltende Säule aufgebraucht; die Säule wird mit 20 l entmineralisiertem Wasser gewaschen und mit wäßriger 2,0 m Natriumchloridlösung eluiert, die mit Ammoniak auf pH 8,5 eingestellt ist. Die erhaltenden 47 l aktive Eluate werden auf eine 3 l Diaion[R] HP-20 (polymeres Adsorptionsmaterial, Fa. Mitsubishi Chemical Industries, Japan) enthaltende Säule aufgebracht; die Säule wird mit 7 l entmineralisiertem Wasser gewaschen und mit Methanol eluiert. Die aktiven Methanoleluate werden im Vakuum eingeengt und die so erhaltene wäßrige Lösung wird mit destilliertem Wasser verdünnt. Der pH wird mit HCl auf 3,0 eingestellt und die Lösung auf eine 2 l Diaion HP-20 enthaltende Säule aufgebracht. Die Säule wird mit entmineralisiertem Wasser gespült, bis das Spülwasser frei von Chloridionen ist. Das Cammunocin wird mit Methanol eluiert; die Methanoleluate werden im Vakuum eingeengt und lyophilisiert. Auf diese Weise erhält man 21 g des antibiotischen Rohkomplexes.

Der antibiotische Rohkomplex wird in bidestilliertem Wasser, pH 1,7 mit Ammoniak, gelöst in zwei gleiche Volumina aufgeteilt und auf zwei Sephadex[R] LH-20 in bidestilliertem Wasser enthaltende, 6,4 x 84 cm große Säulen aufgebracht. Die Säulen werden mit bidestilliertem Wasser mit einer Durchflußmenge von 0,5 ml pro Minute eluiert und das Eluat in 20 ml Fraktionen gesammelt. Insgesamt werden 1,1 l der aktiven Eluate lyophilisiert, wobei man 8 g des antibiotischen Komplexes erhält. Dieses Material wird durch erneutes Chromatographieren auf einer Sephadex[R] LH-20 enthaltenden Säule in oben beschriebener Weise weiter gereinigt, wobei man 4,45 g halbreinen antibiotischen Komplex erhält.

Dieser halbreine antibiotische Komplex wird in 200 ml bidestilliertem Wasser gelöst und auf eine DEAE-Sephadex[R] A-25 (Ionenaustauscher mit Diethylaminoethyl-Funktionalität) in bidestilliertem Wasser enthaltende Säule (6,2 x 26 cm) aufgebracht. Die Säule wird mit Wasser gespült und dann mit einer wäßrigen NaCl-Lösung, wobei deren Molarität durch einen Stufengradienten in 5 %-Schritten gesteigert wird, eluiert. Der antibiotische Komplex wird in 6 l einer 1,5 m NaCl-Lösung und 5 l einer 2 m NaCl-Lösung eluiert. Die kombinierten Eluate, deren pH mit HCl auf 2,0 eingestellt worden war, werden dann auf eine 2 l Diaion[R] HP-20 enthaltende Säule aufgebracht; die Säule wird so lange mit Wasser gespült, bis das Spülwasser keine Chloridionen mehr aufweist und anschließend mit Methanol eluiert. Das Methanol wird im Vakuum abdestilliert und die verbleibende wäßrige Lösung lyophilisiert, wobei man 700mg reines Cammunocin erhält.

## Beispiel 6

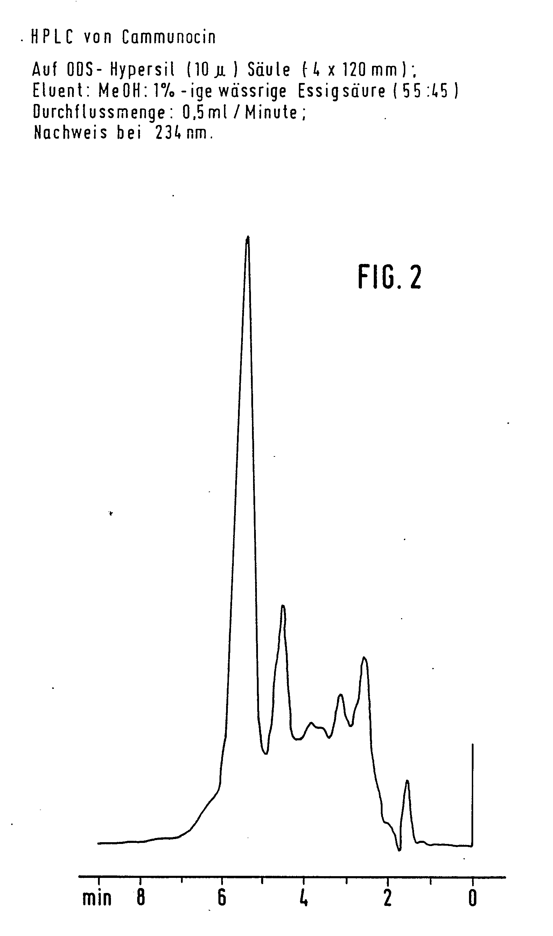

Die HPLC des reinen antibiotischen Komplexes Cammunocin auf einer ODS-Hypersil[R] (10 μ) enthaltenden, 4 x 120 mm großen Säule unter Verwendung eines Eluentgemisches bestehend aus MeOH und 1 % wäßriger Essigsäure (55:45) bei einer Durchflußmenge von 0,5 ml pro Minute und Nachweis bei 234 nm zeigt, daß Cammunocin eine mikroheterogene Mischung ist, d.h. ein Komplex miteinander verwandter antibiotischer Verbindungen, wie Figur 2 zeigt.

Cammunocin wird mit 6 n HCl hydrolysiert und das so erhaltene Hydrolysat nach Methylierung und Trifluoracetylierung mittels GC-EIMS (Gaschromatographie-Elektronenstoßmassenspektrometrie) und GC-CIMS (Gaschromatographie-Massenspektroskopie mit chemischer Ionisierung) unter Verwendung eines Gaschromatographen Perkin Elmer mit 3 % OV-1 auf einer Gaschromon-Q-Säule mit angeschlossenem Massenspektrometer AEI MS-9025 mit einem on-line Datensystem DS-50 SM analysiert. Dabei zeigt sich die Anwesenheit folgender Aminosäuren:

Hauptbestandteile:

α-Aminoadipinsäure
Asparginsäure
Glycin
4-Hydroxyphenylglycin
Serin

Nebenbestandteile:

Glutaminsäure
3-Hydroxyasparaginsäure
Leucin
N-Methylphenylglycin
Prolin
Threonin

Aus den obigen Daten läßt sich der Schluß ziehen, daß Cammunocin ein peptidantibiotischer Komplex ist.

## Ansprüche

1. Cammunocin, gekennzeichnet durch UV-Spektrum in Methanol nach Figur 3 mit Banden bei ca. 234 nm, 280 nm, und 340 nm und IR-Spektrum in KBr nach Figur 4 mit Banden bei ca. 3400 cm$^{-1}$, 1680 cm$^{-1}$, 1559 cm$^{-1}$, 1250 cm$^{-1}$ und 600 cm$^{-1}$.

2. Cammunocin nach Anspruch 1, erhältlich durch Streptomyces species Y-84,36210 (DSM 4329).

3. Verfahren zur Herstellung einer Verbindung gemäß Anspruch 1 oder 2, dadurch gekennzeichnet, daß man Streptomyces species Y-84,36210 (DSM4329) oder einen seiner Varianten oder Mutanten unter aeroben Bedingungen in einem Nährmedium, das Kohlenstoff- und Stickstoffquellen und Nährsalze sowie gegebenenfalls Spurenelemente enthält, kultiviert.

4. Verfahren nach Anspruch 3, dadurch gekennzeichnet, daß die Kultivierung bei einer Temperatur zwischen ca. 18 und 37°C und einem pH-Wert von ca. 6 - 9 durchgeführt wird.

5. Verfahren nach Anspruch 3 oder 4, dadurch gekennzeichnet, daß bei einer Temperatur von ca. 26 - 28°C und einem pH-Wert von ca. 6,4 - 6,6 kultiviert wird.

6. Verfahren nach Anspruch 5, dadurch gekennzeichnet, daß ca. 40 - 45 Stunden lang kultiviert wird.

7. Verfahren nach einem oder mehreren der Ansprüche 3 - 6, dadurch gekennzeichnet, daß submers kultiviert wird.

8. Arzneimittel, gekennzeichnet durch einen Gehalt an Cammunocin gemäß Anspruch 1 oder 2 neben üblichen Hilfs-und/oder Trägerstoffen.

9. Verwendung von Cammunocin gemäß Anspruch 1 oder 2 zur Herstellung von Arzneimitteln mit antibiotischer und/oder immunsuppressiver Wirkung.

10. Streptomyces species Y-84,36210 (DSM 4329).

Patentansprüche für folgenden Vertragsstaat: GR

1. Verfahren zur Herstellung von Cammunocin, dadurch gekennzeichnet, daß man Streptomyces species Y-84,36210 (DSM4329) oder einen seiner Varianten oder Mutanten unter aeroben Bedingungen in einem Nährmedium, das Kohlenstoff- und Stickstoffquellen und Nährsalze sowie gegebenenfalls Spurenelemente enthält, kultiviert und das Cammunocin aus der Kulturbrühe isoliert und reinigt.

2. Verfahren nach Anspruch 1, dadurch gekennzeichnet, daß die Kultivierung bei einer Temperatur zwischen ca. 18 und 37° C und einem pH-Wert von ca. 6 - 9 durchgeführt wird.

3. Verfahren nach Anspruch 1 oder 2, dadurch gekennzeichnet, daß bei einer Temperatur von ca. 26 - 28° C und einem pH-Wert von ca. 6,4 - 6,6 kultiviert wird.

4. Verfahren nach einem oder mehreren der Ansprüche 1-3, dadurch gekennzeichnet, daß ca. 40 - 45 Stunden lang kultiviert wird.

5. Verfahren nach einem oder mehreren der Ansprüche 1 - 4, dadurch gekennzeichnet, daß submers kultiviert wird.

6. Verfahren zur Herstellung von Arzneimitteln, dadurch gekennzeichnet, daß Cammunocin, hergestellt gemäß einem oder mehreren der vorausgehenden Ansprüche, mit üblichen Hilfs- und/oder Trägerstoffen in eine geeignete Darreichungsform gebracht wird.

7. Verwendung von Cammunocin zur Herstellung von Arzneimitteln mit antibiotischer und/oder immunsuppressiver Wirkung.

9. Streptomyces species Y-84,36210 (DSM 4329).


Patentansprüche für folgenden Vertragsstaat: ES

1. Verfahren zur Herstellung von Cammunocin, dadurch gekennzeichnet, daß man Streptomyces species Y-84,36210 (DSM4329) oder einen seiner Varianten oder Mutanten unter aeroben Bedingungen in einem Nährmedium, das Kohlenstoff- und Stickstoffquellen und Nährsalze sowie gegebenenfalls Spurenelemente enthält, kultiviert und das Cammunocin aus der Kulturbrühe isoliert und reinigt.

2. Verfahren nach Anspruch 1, dadurch gekennzeichnet, daß die Kultivierung bei einer Temperatur zwischen ca. 18 und 37° C und einem pH-Wert von ca. 6 - 9 durchgeführt wird.

3. Verfahren nach Anspruch 1 oder 2, dadurch gekennzeichnet, daß bei einer Temperatur von ca. 26 - 28° C und einem pH-Wert von ca. 6,4 - 6,6 kultiviert wird.

4. Verfahren nach einem oder mehreren der Ansprüche 1-3, dadurch gekennzeichnet, daß ca. 40 - 45 Stunden lang kultiviert wird.

5. Verfahren nach einem oder mehreren der Ansprüche 1 - 4, dadurch gekennzeichnet, daß submers kultiviert wird.

6. Verfahren zur Herstellung von Arzneimitteln, dadurch gekennzeichnet, daß Cammunocin, hergestellt gemäß einem oder mehreren der vorausgehenden Ansprüche, mit üblichen Hilfs- und/oder Trägerstoffen in eine geeignete Darreichungsform gebracht wird.

7. Verwendung von Cammunocin zur Herstellung von Arzneimitteln mit antibiotischer und/oder immunsuppressiver Wirkung.

# FIG.1

DC von Cammunocin.
Lösungsmittelsystem: EtOAc : MeOH : H$_2$O (4:4:1)

HPLC von Cammunocin

Auf ODS- Hypersil (10 μ) Säule (4 x 120 mm);
Eluent: MeOH: 1% -ige wässrige Essigsäure (55:45)
Durchflussmenge: 0,5 ml / Minute;
Nachweis bei 234 nm.

FIG. 2

min  8      6      4      2      0

UV-Spektrum von Cammunocin in MeOH

FIG. 3

Infrarotspektrum von Cammunocin in KBr

FIG.4

Europäisches
Patentamt

**EUROPÄISCHER RECHERCHENBERICHT**

Nummer der Anmeldung

EP 89 10 1691

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | * Keine Entgegenhaltungen *<br>----- | | C 12 P   1/06<br>C 07 G  11/00<br>A 61 K  35/66 //<br>(C 12 P   1/06<br>C 12 R   1:465) |
| | | | **RECHERCHIERTE SACHGEBIETE (Int. Cl.4)**<br><br>C 12 P<br>C 12 R<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 17-05-1989 | RAJIC M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.........................................................................................
& : Mitglied der gleichen Patentfamilie, übereinstimmendes Dokument

EPO FORM 1503 03.82 (P0403)